# EUROPEAN PATENT SPECIFICATION

(11) **EP 0 361 682 B1**
(45) Date of publication and mention of the grant of the patent: **27.03.1996**
(21) Application number: 89308687.6
(22) Date of filing: 29.08.1989
(51) Int. Cl.: G03F 7/029, C07D 251/22, C07D 401/14

(54) **Halomethyl-1,3,5-triazines containing a photoinitiator moiety**
Photoinitiator enthaltende Halogenmethyl-1,3,5-Triazine
Halométhyl 1,3,5 triazine comportant un groupe photoinitiateur

(30) Priority: 07.09.1988 US 241339
(43) Date of publication of application: 04.04.1990
(73) Proprietor: MINNESOTA MINING AND MANUFACTURING COMPANY, St. Paul, Minnesota 55133-3427 (US)
(72) Inventor: Rossman, Mitchell A. c/o Minnesota Mining and, St.Paul - Minnesota 55144-1000 (US); Bonham, James A. c/o Minnesota Mining and, St.Paul - Minnesota 55144-1000 (US)
(74) Representative: Baillie, Iain Cameron

(56) References cited:
- EP-A- 0 305 115
- US-A- 4 476 215

## Description

### Field of the Invention

This invention relates to photosensitive compounds, more particularly, derivatives of halomethyl-1,3,5-triazines.

### Discussion of the Prior Art

Compounds that decompose to generate free radicals (free radical generating agents) upon exposure to light are well known in the graphic arts. Organic halogen compounds, which are capable of generating free radicals such as a chlorine free radical or a bromine free radical upon exposure to light, have been widely used as photoinitiators in photopolymerizable compositions, as photoactivators in free radical photographic compositions, and as photoinitiators for reactions catalyzed by acids formed by light. The spectral sensitivity of these compositions may be broadened by the addition of sensitizers which, in essence, transfer their absorbed energy to the organic halogen compound. The use of such halogen compounds in photopolymerization processes and free radical photographic processes has been described in Kosar, Light-Sensitive Systems, J. Wiley & Sons (New York, 1965), pp. 180-181, 361-370.

Halomethyl-1,3,5-triazines are known to be initiators for a number of photochemical reactions. They are employed to produce free radicals for initiating polymerization or color changes and for initiating secondary reactions upon liberation of acid by the interaction of the free-radicals when hydrogen donors are present.

Examples of the use of the halomethyl-1,3,5-triazines in the free radical polymerization of acrylate monomers are described in U.S. Patent No. 3,905,815; U.S. Patent No. 3,617,288; U.S. Patent No. 4,181,752; U.S. Patent No. 4,391,687; U.S. Patent No. 4,476,215; and DE 3,517,440. U.S. Patent No. 3,779,778 discloses the photoinitiated acid catalyzed decomposition of pyranyl ether derivatives to produce photosolubilizable compositions useful as positive printing plates. Chromophore substituted styryl-1,3,5-triazines and their uses are disclosed in U.S. Patent No. 3,987,037 and U.S. Patent No. 3,954,475.

Radiation sensitive compositions containing biand polyaromatic substituted triazines are disclosed in U.S. Patent No. 4,189,323.

Typical photopolymerization initiators for polymerizable ethylenically unsaturated compounds include benzil, benzoin, benzoin ethyl ether, Michler's ketone, anthraquinone, acridine, phenazine, benzophenone, etc. For example, the use of photoinitiators such as benzoin ethers are described in U.S. Patent No. 2,722,512; anthraquinones are described in U.S. Patent No. 3,046,127; amino phenyl ketones and active ethylene or amino compounds are described in U.S. Patent No. 3,661,588; and Michler's ketone and benzophenone are described in U.S. Patent No. 3,682,641.

Mixed photoinitiators have been disclosed for use with polymerizable monomers. A very effective mixed initiator system has been Michler's ketone admixed with benzophenone, as described in U.S. Patent No. 3,682,641. 3-Keto-coumarins have been shown to be effective mixed initiators with Michler's ketone, as described in U.S. Patent No. 4,366,228. Halogenated organic photoinitiators such as carbon tetrabromide, etc, have been used as co-photoinitiators with diazonium salts, as described in U.S. Patent No. 4,113,497. Likewise, 5-isoxazolones are effective co-photoinitiators when used with aromatic carbonyl compounds in the presence of halomethyl-1,3,5-triazines, as described in U.S. Patent No. 4,254,432. Other examples of specific combinations of known photoinitiators are disclosed in U.S. Patent Nos. 3,814,607; 3,673,140; 3,326,710; 3,847,771; 3,427,161; 3,915,824, and 4,264,709.

### SUMMARY OF THE INVENTION

This invention provides radiation-sensitive organo-halogen compounds having good sensitivity in the ultraviolet and visible range of the spectrum. These compounds are suitable for use in radiation-sensitive compositions. This invention provides compounds that have a photo-labile halomethyl-1,3,5-triazine moiety and an additional photoinitiator moiety within one molecule so as to eliminate the need for a combination of photoinitiator compounds. The compounds of this invention are gocd photoinitiators. Photopolymerizable and photocrosslinkable compositions containing these photoinitiators can be used in printing, duplicating, copying, and other imaging systems.

This invention provides a compound having the formula: 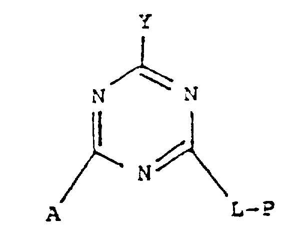 wherein
A represents a member selected from the group consisting of mono-, di- and trihalomethyl groups,
Y represents a member selected from the group consisting of A, L-P, NH₂, NHR, NR₂, OR, and R', where R independently represents a substituted or unsubstituted alkyl group, or a substituted or unsubstituted aryl group, R' represents a substituted or unsubstituted alkyl group, a substituted or unsubstituted aryl group, a substituted or unsubstituted alkenyl group, or a substituted or unsubstituted heterocyclic aromatic group.
P represents a photoinitiator moiety capable of initiating free radical or ionic chain polymerization upon exposure to actinic radiation, and
L represents a group or a covalent bond linking the photoinitiator moiety to the triazine nucleus, the portion of L directly attached to the triazine nucleus being selected from:
   (a) carbon atom wherein the carbon atom of the portion of said linking group directly attached to the triazine nucleus is a member of the group selected from alkyl groups, aliphatic groups, haloalkyl groups, alkenyl groups, aryl groups, styryl groups, ester groups (-CO₂-), and combinations of the foregoing;
   (b) amino group selected from, (1) aminoaryl groups wherein the nitrogen atom of the amino group is attached to the triazine nucleus, and (2) an amino group wherein the nitrogen atom of the amino group is attached to both the triazine nucleus and the carbon atom of at least one -CH₂CH₂- group; and
   (c) oxygen atom.
Preferably A is a trihalomethyl group. Representative examples of photoinitiator moieties P include benzoin group, benzoin alkyl ether group, acetophenone group, dialkoxyacetophenone group, benzophenone group, fluorenone group, anthraquinone group, thioxanthone group, triarylsulfonium group, dialkylarylsulfonium group, diaryliodonium group,

Preferably R represents a substituted or α-acyloxime group, azide group, diazonium group, 3-ketocoumarin group, bisimidazole group, or a halomethyl-1,3,5-triazine group covalently bonded to the first-mentioned halomethyl-1,3,5-triazine moiety. The compound is capable of being stimulated by actinic radiation at a wavelength of 220 to 900 nanometers, whereby free radicals or acids or both are generated.

These compounds are useful as photoinitiators for photosensitive compositions and elements. Thus, they can be incorporated in photopolymerizable compositions and printing compositions useful for producing printing plates, such as lithographic plates, relief plates or gravure plates, photoresists and photographic elements, and photosensitive resist forming compositions with which visible images can be obtained upon exposure to light.

### DETAILED DESCRIPTION OF THE INVENTION

As used herein, the term "photoinitiator moiety" means a moiety containing at least one group that is capable of initiating free radical or ionic chain polymerization upon exposure to actinic radiation.

Halomethyl-1,3,5-triazine compounds of this invention can as mentioned be represented by the general formula I: 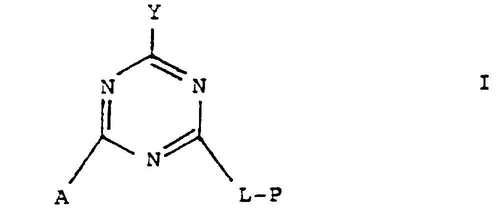 wherein A, P and L are as defined above.

Preferably R represents a substituted or unsubstituted alkyl group having 1 to 6 carbon atoms. Preferably R' represents a substituted or unsubstituted alkyl group having 1 to 6 carbon atoms, or a substituted or unsubstituted alkenyl group having 2 to 6 carbon atoms.

Halomethyl groups that are suitable for the present invention include chloro-, bromo-, and iodomethyl groups, with chloro- and bromomethyl groups being preferred. Trihalomethyl groups are preferred; most preferred are trichloromethyl and tribromomethyl groups.

Y represents any of a variety of substituents that are useful in modifying the physical, e.g., solubility, or chemical properties of the molecule, and preferably represents A, L-?, or R'. When Y represents A, the maximum number of halomethyl groups per triazine nucleus can be made available for free radical generation. When Y represents L-P, the chemical composition for both L-P groups can be the same, or it can be different, depending on the composition of linking group L, photoinitiator moiety P, or both. When Y represents R', and in particular when R' represents an aryl, aralkenyl, or heterocyclic aromatic group, the spectral sensitivity of the molecule can be varied, based on the photochemical response of R' to actinic radiation.

when R or R' represents an aryl group it is preferred that the group have a maximum or five rings, more preferably three rings, and most preferably one ring.

When R or R' represents a substituted group, the particular identity of the substituents is not critical. However, the substituents should be selected so as not to adversely affect the photoinitiation characteristics or light sensitivity of the compounds of this invention.

P preferably represents at least one group selected from the group consisting of benzoin group, benzoin alkyl ether group, an acetophenone group, dialkoxyacetophenone group, benzophenone group, anthraquinone group, thioxanthone group, triarylsulfonium group, diaryliodonium group, α-acyloxime group, azide group, diazonium group, 3-ketocoumarin group, bisimidazole group, fluorenone group, or a halomethyl-1,3,5-triazine group covalently bonded to the triazine nucleus of formula I. There is no upper limit on the number of photoinitiator moieties per triazine nucleus; there is no upper limit on the number of triazine nuclei per photoinitiator moiety; however, there must be at least one photoinitiator moiety per triazine nucleus. Preferably, the number of photoinitiator moieties per triazine nucleus ranges from one to two or two to one; more preferably, there is one photoinitiator moiety per each triazine nucleus. If more than one photoinitiator moiety is present per triazine nucleus, they can be from different generic classes or can be different species from the same generic class. If more that one triazine nucleus is present per photoinitiator moiety, they can be of different species.

L represents a group that links the photoinitiator moiety or moieties to the triazine nucleus. The precise identity of L is not critical (within the limits mentioned above), but it should be selected so that it does not interfere with or adversely affect the photoinitiation characteristics or light sensitivity of the compound. L can be formed from a single group or it can be formed from a combination of groups. In addition, as mentioned, L also includes a covalent bond. Groups that are suitable for linking groups include amino (-NH-), aliphatic, e.g., having up to 10 carbon atoms, alkyl, e.g., having up to 10 carbon atoms, haloalkyl, e.g., having up to 10 carbon atoms, alkenyl, e.g., having up to 10 carbon atoms, aryl, e.g., having one ring, styryl, ester (-CO₂-), ether (-O-), and combinations thereof. Based on ease of synthesis, the most preferred groups for attachment directly to the triazine nucleus are amino, alkenyl, aryl, and ether.

The following list exemplifies typical -L-P group combinations:

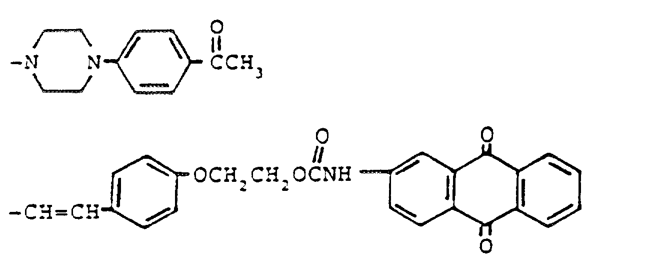

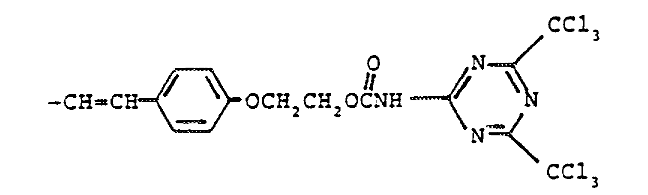

EP-A-0,305,115, which is available as a piece of prior art against the subject invention for the purposes only of Articles 54(3) and 54(4) EPC discloses that one method of preparing certain compounds is by the addition reaction of isocyanato-substituted halomethyl-1,3,5-triazines with photoinitiators having groups reactive with the isocyanate group. The isocyanato substituted triazines may be prepared from the corresponding amino derivative according to the procedure of U. Von Gizycki, Angew. Chem. Int. Ed. Eng., 1971, 10, 403. Isocyanato-1,3,5-triazines suitable for this reaction include:
2,4-bis(trichloromethyl)-6-isocyanato-1,3,5-triazine
2-isocyanato-4-methyl-6-trichloromethyl-1,3,5-triazine
2-isocyanato-4-phenyl-6-trichloromethy1-1,3,5-triazine
2-isocyanato-4-methoxy-6-trichloromethyl
2-isocyanato-4-(p-methoxyphenyl)-6-trichloromethyl 1,3,5-triazine
2-isocyanato-4-(p-methoxystyryl)-6-trichloromethyl-1,3,5-triazine
2-isocyanato-4-(m,p-dimethoxyphenyl)-6-trichloromethyl-1,3,5-triazine

Typical photoinitiators that will combine with the isocyanato group include 1-benzoyl cyclohexanol (Irgacure^{R} 184), 4-hydroxyacetophenone, 4-hydroxybenzophenone, 4-aminobenzophenone, 2-amino-9-fluorenone, 2-aminoanthraquinone, 2-hydroxymethylanthraquinone, 4'-piperidinoacetophenone, 4-hydroxydiphenyliodonium salt, dimethyl-4-hydroxyphenylsulfonium salt, and 2,4-bis(trichloromethyl)-6-hydroxyethylamino-1,3,5-triazine.

The isocyanate addition reaction can be carried out in the presence of solvents such as, for example, toluene, pyridine, benzene, xylene, dioxane, tetrahydrofuran, etc., and mixtures of solvents. The duration and temperature of the reaction is dependent on the particular compounds and the catalyst employed. Generally, temperatures ranging from about 25°C to 150°C for from one to seventy-two hours are sufficient to provide for the reaction. Preferably, the reaction is carried out at room temperature from three to seventy-two hours. The preferred catalyst is di-n-butyltin dilaurate.

Another method for preparing the compounds of this invention is the cotrimerization of organic nitriles having a photoinitiator substituent with haloacetonitriles in accordance with the teachings of Wakabayashi et al, Bulletin of the Chemical Society of Japan, 1969, 42, 2924-30. Still another method is the condensation reaction of an aldehyde compound having a photoinitiator functionality in accordance with the teachings of U.S. Patent No. 3,987,037. Another method is the nucleophilic displacement reactions on halomethyl-1,3,5-triazines using photoinitiators having free hydroxy or amino groups.

The photoinitiators of this invention combine a halomethyl-1,3,5,-triazine moiety and another photoinitiator moiety in the same molecule, thereby eliminating the requirement of adding each type of photoinitiator separately. Synergism can be brought about in instances where the radicals produced by one moiety induce chain decomposition of the other moiety, thereby in effect, promoting the same reaction that would have been produced by a direct photoresponse.

Combinations of moieties can be selected that produce, upon exposure to actinic radiation, several different photoproducts, which will initiate the same reaction. For example, a halomethyl-1,3,5,-triazine moiety combined with a benzoin group can initiate free radical polymerization by generating halogen radicals and benzoyl radicals (along with other unidentified radical species).

Combinations of moieties can be selected that produce photoproducts which will initiate a multiplicity of reactions. For example, compounds derived from a halomethyl-1,3,5,-triazine and a sulfonium salt can produce both free radicals and Lewis acids to cure a mixture comprising acrylate and epoxy monomers.

Furthermore, the chromophore of each photoinitator moiety can be selected so that each moiety can either (a) broadly respond to radiation, thereby allowing more efficient utilization of various light sources commercially available, or (b) narrowly respond to different wavelengths, thereby allowing stepwise initiation of multiple reactions. An example of the latter scheme would be to first irradiate the compound at a wavelength at which the halomethyl-1,3,5-triazine moiety absorbs light to initiate a free radical polymerization, and then subsequently irradiate the compound at a wavelength at which the other photoinitiator moiety absorbs light to initiate an ionic cross-linking reaction.

The sensitivity of compositions containing the compounds of this invention to actinic radiation of a particular range of wavelengths can be increased by the incorporation of ultraviolet and visible light sensitizers, such as, for example, cyanine, carbocyanine, merocyanine, styryl, acridine, polycyclic aromatic hydrocarbons, polyarylamines and amino-substituted chalcones. Suitable cyanine dyes are described in U.S. Patent No. 3,495,987. Suitable styryl dyes and polyarylamines are described in Kosar, Light Sensitive Systems, J. Wiley and Sons (New York, 1965), pp 361-369. Polycyclic aromatic hydrocarbons useful as sensitizers, e.g. 2-ethyl-9,10-dimethoxyanthracene, are disclosed in U.S. Patent No. 3,640,718. Amino substituted chalcones useful as sensitizers are described in U.S. Patent No. 3,617,288. The compounds of this invention can be used in photosensitive compositions in combination with other photoinitiators including the benzophenones, benzoin ethers, thioxanthone, benzil, and Michler's ketone. The compounds of this invention can be substituted for the triazines used in conjunction with dialkylamino aromatic carbonyl compounds disclosed in U.S. Patent No. 4,259,432, with 2-(benzoylmethylene)-5-benzothiazolidene thiazole-4-1 compounds disclosed in E application 0109291, May 23, 1984, with 3-keto-substituted coumarin compounds disclosed in U.S. Patent No. 4,505,793, with those described in U.S. Patent No. 4,239,850, Jpn. Kokai Tokkyo Koho JP 60 60,104 (85 60104), and Ger. Offen 2,851,641.

The photoinitiators of this invention can be used with photopolymerizable compositions comprising unsaturated, free radical initiated, chain propagating addition polymerizable compound, the photoinitiator of this invention, and, optionally, one or more fillers, binders, dyes, polymerization inhibitors, color precursors, oxygen scavengers, etc. The photoinitiators of this invention should be present in an amount sufficient to initiate polymerization of the polymerizable compound. For every 100 parts of polymerizable compound, there can be present from 0.005 to 10 parts of photoinitiator, from 0 to 200 parts of filler, from 0 to 200 parts of binder*,* and from 0 to 10 or more parts of dyes, polymerization inhibitors, color precursors, oxygen scavengers, etc., as may be needed for a particular use of the photopolymerizable composition. Preferably, per each 100 parts of polymerizable compounds, there are from 1 to 7.5 parts of photoinitiator and from 25 to 150 parts of binder.

Unsaturated, free-radical initiated, chain-propagating addition polymerizable compounds suitable for the compositions of this invention include alkylene or polyalkylene glycol diacrylates, e.g., ethylene glycol diacrylate, diethylene glycol diacrylate, glycerol diacrylate, glycerol triacrylate, ethylene glycol dimethacrylate, 1,3-propanediol dimethacrylate, 1,2,4-butanetriol trimethacrylate, 1,4-cyclohexanediol diacrylate, pentaerythritol tetramethacrylate, pentaerythritol triacrylate, sorbitol hexacrylate; bis[1-(3-acryloxy-2-hydroxy)]-p-propoxyphenyl dimethylmethane, bis[1-(2-acryloxy)]-p-ethoxyphenyl-dimethylmethane, tris hydroxyethyl-isocyanurate trimethacrylate, the bis-acrylate and the bis-methacrylates of polyethylene glycols of molecular weight 200-500 and the like; unsaturated amides, e.g., methylene bis-acrylamide, methylene bis-methacrylamide, 1,6-hexamethylene bis-acrylamide, diethylene triamine trisacrylamide, beta-methacrylaminoethyl methacrylate; vinyl esters such as divinyl succinate, divinyl adipate, divinyl phthalate. The preferred unsaturated compounds inciude pentaerythritol tetracrylate, his[p-(3-acryloxy-2-hydroxypropoxy)phenyl] dimethylmethane, and bis[p-(2-acryloxyethoxy)phenyl] dimethylmethane. Mixtures of these esters can be used as well as mixtures of these esters with alkyl esters of acrylic acid and methacrylic acid, including such esters as methyl acrylate, methyl methacrylate, ethyl acrylate, isopropyl methacrylate, n-hexyl acrylate, stearyl acrylate, allyl acrylate and diallyl phthalate.

To prepare the photosensitive compositions of this invention, the components can be admixed in any order and stirred or milled to form a solution or uniform dispersion. Photosensitive elements can be made by coating the photosensitive composition on a suitable base or support and drying the coating. The dry thickness of the coating ranges typically from 0.000127 to 0.19 cms (0.00005 to 0.075 inch).

Bases or supports for the photosensitive compositions include metals, e.g., steel and aluminum plates, sheets and foils, and films or plates composed of various film-forming synthetic or high polymers, including addition polymers, e.g. vinylidene chloride, vinyl chloride, vinyl acetate, styrene, isobutylene polymers and copolymers; linear condensation polymers e.g., polyethylene terephthalate, polyhexamethylene adipate, polyhexamethylene adipamide/adipate.

The invention will be more specifically illustrated by the following examples. All values of Xmax were measured in tetrahydrofuran, unless otherwise indicated.

### Example 1

To a solution of 1 equivalent 2-amino-4,6-bis(trichloromethyl)-1,3,5-triazine in dry toluene at 0°C was added dropwise over a 15 minute period a solution of trichloroacetyl chloride in dry toluene. The resulting solution was heated at reflux under nitrogen atmosphere for three hours and cooled to room temperature. The solvent was reduced by means of a rotary evaporator under reduced pressure. The precipitate was filtered and dried to afford product. The product had a melting point of 101-108°C and a Xmax of 239 nm. The structure of the product is shown below.

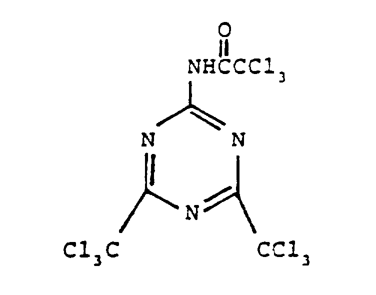

### Example 2

The procedure of Example 1 was repeated, with the only exception being that N,N'-bis-[2,4-bis(trichloromethyl)-1,3,5-triazinyl-(6)]-ethylenediamine was used instead of 2-amino-4,6-bis(trichloromethyl)-1,3,5-triazine. The product had a melting point of 209-213°C and a Xmax of 274 nm. The structure of the product is shown below.

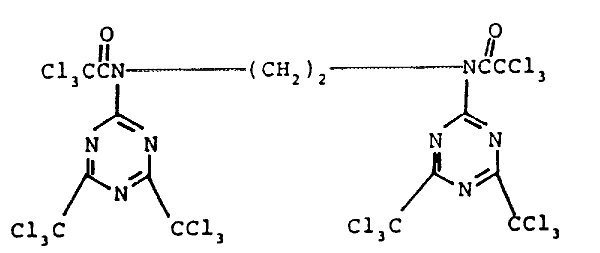

### Example 3

The procedure of Example 1 was repeated, with the only exception being that 2,6-bis-[2,4-bis(trichloromethyl)-1,3,5-triazinyl-(6)-amino]pyridine was used instead of 2-amino-4,6-bis(trichloromethyl)-1,3,5-triazine. The product had a melting point of 243-248°C and a Xmax of 324 nm, 233 nm. The structure of the product is shown below.

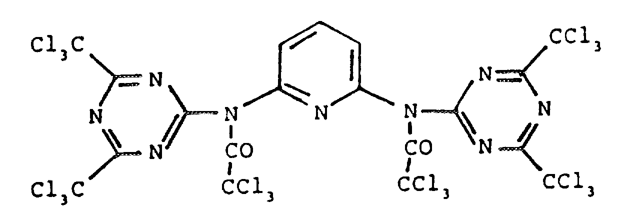

### Example 4

To a solution of 2.3 mmol 2,4,6-tris(trichloromethyl)-1,3,5-triazine in 25 ml toluene was added 1 equivalent 4'-piperazinoacetophenone. The reaction mixture was stirred at room temperature for 24 hours under nitrogen atmosphere. The solvent was removed by means of a rotary evaporator under reduced pressure, and the residue was dissolved in a small amount of dichloromethane and loaded upon a column of silica gel (100 g packed in hexane) and eluted with hexane. The appropriate fractions were pooled and the solvent was removed by means of a rotary evaporator to afford product. The product had a melting point of 152-155°C and a Xmax of 312 nm, 240 nm. The structure of the product is shown below.

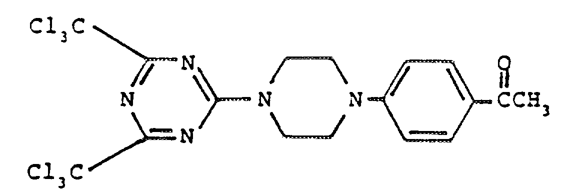

### Example 5

This example illustrates the preparation of photosensitive elements using the halomethyl-1,3,5-triazines of the present invention.

A solution was prepared from 74.24 g azeotrope of 1-propanol and water (71.8% 1-propanol/28.2% water), 4.32 g pentaerythritol tetraacrylate ("Sartomer" monomer SR-295, Arco Chemical Company), 5.64 g oligomer (prepared according to U.S. Patent No. 4,228,232 and 60.9% in methyl ethyl ketone), 0.30 g triethylamine, and 14.88 g a 1:1 mixture of polyvinyl acetate-methylal resin ("Formvar" 12/85T, Union Carbide Corp.) and red pigment (Pigment Red 48, C.I. 15865) (9.4% by weight solution of the azeotrope). To 2.5 g of this solution was added 2.5 mg dimethylaminobenzylacetone (DMBA), 10 mg initiator, and the resulting solution shaken in the dark for 15 minutes. The solution was filtered through glass wool and coated onto a grained, anodized aluminum plate with a #12 Mayer bar. The plate was dried at 66°C for 2 min and cooled to room temperature. To this was applied a topcoat formulation (prepared from 5.00 g carboxymethyl cellulose ether (CMC-7L), 0.26 g surfactant ("Triton" X-100 (10% in water)), and 95 g water with a #14 Mayer bar and carefully dried with a heat gun. The plates were exposed for 5 sec in air on top of a draw-down glass in a 3M Seventy exposure unit equipped with a 2 kw photopolymer bulb through a √2, 21 step Stouffer step tablet. The plates were soaked in the developer solution prepared from 784.40 g deionized water, 16.70 g sodium metasilicate pentahydrate, 33.40 g 1-propanol, and 0.50 g surfactant ("Dowfax-2A1", Dow Chemical Company) (45% solution in water) for 15 sec and rubbed 10 times with a 4 in. x 4 in. cotton pad. The relative sensitivities for triazines of Examples 1-3 are shown in Table 2.

**Table 2**

| Initiator | Solid Step |
|---|---|
| Example 1 | 13 |
| Example 2 | 13 |
| Example 3 | 13 |

## Claims

1. A compound having the formula: 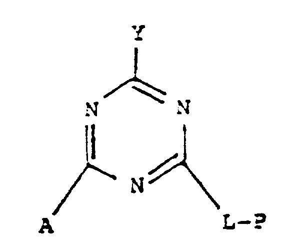 wherein
A represents a member selected from the group consisting of mono-, di- and trihalomethyl groups,
Y represents a member selected from the group consisting of A, L-P, NH₂, NHR, NR₂, OR, and R', where R independently represents a substituted or unsubstituted alkyl group, or a substituted or unsubstituted aryl group, R' represents a substituted or unsubstituted alkyl group, a substituted or unsubstituted aryl group, a substituted or unsubstituted alkenyl group, or a substituted or unsubstituted heterocyclic aromatic group.
P represents a photoinitiator moiety capable of initiating free radical or ionic chain polymerization upon exposure to actinic radiation, and
L represents a group or a covalent bond linking the photoinitiator moiety to the triazine nucleus, the portion of L directly attached to the triazine nucleus being selected from:
(a) carbon atom wherein the carbon atom of the portion of said linking group directly attached to the triazine nucleus is a member of the group selected from alkyl groups, aliphatic groups, haloalkyl groups, alkenyl groups, aryl groups, styryl groups, ester groups (-CO₂-), and combinations of the foregoing;
(b) amino group selected from, (1) aminoaryl groups wherein the nitrogen atom of the amino group is attached to the triazine nucleus, and (2) an amino group wherein the nitrogen atom of the amino group is attached to both the triazine nucleus and the carbon atom of at least one -CH₂CH₂- group; and
(c) oxygen atom.

2. The compound of claim 1 wherein A represents a trihalomethyl group.

3. The compound of claim 2 wherein the trihalomethyl group is a member selected from trichloromethyl, tribromomethyl, triiodomethyl.

4. The compound of claim 3 wherein the trihalomethyl group is a member selected from trichloromethyl and tribromomethyl.

5. The compound of any preceding claim wherein the photoinitiator moiety is selected from benzoin group, benzoin alkyl ether group, acetophenone group, dialkoxyacetophenone group, benzophenone group, fluorenone group, anthraquinone group, thioxanthone group, triarylsulfonium group, diaryliodonium group, α-acyloxime group, azide group, diazonium group, 3-ketocoumarin group, bisimidazole group, and halomethyl-1,3,5-triazine group.

6. The compound of any preceding claim wherein Y represents A.

7. The compound of any one of claims 1-5 wherein Y represents L-P.

8. The compound of any preceding claim wherein R' represents a substituted or unsubstituted aryl group.

9. The compound of any preceding claim wherein R' represents a substituted or unsubstituted heterocyclic aromatic group.

10. The compound of any one of claims 1-8 wherein R' represents a substituted or unsubstituted alkenyl group.

11. A radiation-sensitive composition comprising:
(1) an ethylenically unsaturated, polymerizable compound, and
(2) a compound according to any preceding claim.

## Patentansprüche

1. Verbindung der Formel: 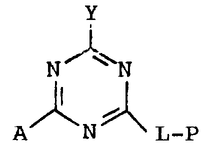 worin sind:
A ein Vertreter, ausgewählt aus der Gruppe, bestehend aus Mono-, Di- und Trihalogenmethyl-Gruppen;
Y ein Vertreter, ausgewählt aus der Gruppe, bestehend aus A, L-P, NH₂, NHR, NR₂, CR und R', worin R unabhängig eine substituierte oder nichtsubstituierte Alkyl-Gruppe oder eine substituierte oder nichtsubstituierte Aryl-Gruppe darstellt, R' eine substituierte oder nichtsubstituierte Alkyl-Gruppe oder eine substituierte oder nichtsubstituierte Aryl-Gruppe, eine substituierte oder nichtsubstituierte Alkenyl-Gruppe oder eine substituierte oder nichtsubstituierte heterocyclische aromatische Gruppe darstellt;
P ein Photoinitiator-Teil, der bei Exponierung an aktinischer Strahlung eine Radikalkettenpolymerisation oder eine Ionenkettenpolymerisation starten kann; sowie
L eine Gruppe oder eine kovalente Bindung, die den Photoinitiator-Teil mit dem Triazin-Ring verknüpft, wobei der direkt an dem Triazin-Ring gebundene Abschnitt L ausgewählt wird aus:
(a) Kohlenstoff-Atom, wobei das Kohlenstoff-Atom des Abschnittes der direkt am Triazin-Ring gebundenen, verknüpfenden Gruppe ein Vertreter der Gruppe ist, ausgewählt aus Alkyl-Gruppen, aliphatischen Gruppen, Halogenalkyl-Gruppen, Alkenyl-Gruppen, Aryl-Gruppen, Styryl-Gruppen, Ester-Gruppen, (-COO-) und Kombinationen davon;
(b) Amino-Gruppen, ausgewählt aus (1) Aminoaryl-Gruppen, bei denen das Stickstoff-Atom der Amino-Gruppe an dem Triazin-Ring gebunden ist, und (2) einer Amino-Gruppe, bei der das Stickstoff-Atom der Amino-Gruppe sowohl an dem Triazin-Ring als auch an dem Kohlenstoff-Atom mindestens einer -CH₂CH₂-Gruppe gebunden ist; sowie (c) Sauerstoff-Atom.

2. Verbindung nach Anspruch 1, bei welcher A eine Trihalogenmethyl-Gruppe darstellt.

3. Verbindung nach Anspruch 2, bei welcher die Trihalogenmethyl-Gruppe ein Vertreter ist, ausgewählt aus Trichlormethyl, Tribrommethyl, Triiodmethyl.

4. Verbindung nach Anspruch 3, bei welcher die Trihalogenmethyl-Gruppe ein Vertreter ist, ausgewählt aus Trichlormethyl, Tribrommethyl.

5. Verbindung nach einem der vorgenannten Ansprüche, bei welchem der Photoinitiator-Teil ausgewählt aus den Gruppen: Benzoin, Benzoinalkylether, Azetophenon, Dialkoxyazetophenon, Benzophenon, Fluorenon, Anthrachinon, Thioxanthon, Triarylsulfonium, Diaryliodonium, «-Acyloxim, Azid, Diazonium, 3-Ketocumarin, Bisimadazol und Halogenmethyl-1,3,5-triazin.

6. Verbindung nach einem der vorgenannten Ansprüche, bei welcher Y dargestellt wird durch A.

7. Verbindung nach Anspruch 1 bis 5, bei welcher Y dargestellt wird durch L-P.

8. Verbindung nach einem der vorgenannten Ansprüche, bei welcher R' eine substituierte oder nichtsubstituierte Aryl-Gruppe darstellt.

9. Verbindung nach einem der vorgenannten Ansprüche, bei welcher R' eine substituierte oder nichtsubstituierte heterocyclische aromatische Gruppe darstellt.

10. Verbindung nach Anspruch 1 bis 8, bei welcher R' eine substituierte oder nichtsubstituierte Alkenyl-Gruppe darstellt.

11. Strahlungsempfindliche Zusammensetzung, umfassend eine:
(1) ethylenisch ungesättigte polymerisierbare Verbindung, und
(2) eine Verbindung nach einem der vorgenannten Ansprüche.

## Revendications

1. Composé ayant la formule : 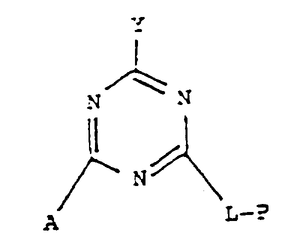 dans laquelle :
A représente un membre choisi dans le groupe comprenant les groupes mono-, di- et trihalométhyle,
Y représente un membre choisi dans le groupe comprenant A, L-P, NH₂, NHR, NR₂, OR et R', où R représente indépendamment un groupe alkyle substitué ou non substitué ou bien un groupe aryle substitué ou non substitué, R' représente un groupe alkyle substitué ou non substitué, un groupe aryle substitué ou non substitué, un groupe alcényle substitué ou non substitué ou un groupe aromatique hétérocyclique substitué ou non substitué,
P représente un fragment photoinitiateur pouvant initier une polymérisation radicalaire ou par chaîne ionique lors d'une exposition à un rayonnement actinique, et
L représente un groupe ou une liaison covalente reliant le fragment photoinitiateur au noyau de triazine, la partie du L directement attachée au noyau de triazine étant choisie parmi :
(a) un atome de carbone, l'atome de carbone de la partie du groupe de liaison directement attachée au noyau de triazine étant un membre du groupe comprenant les groupes alkyle, les groupes aliphatiques, les groupes haloalkyle, les groupes alcényle, les groupes aryle, les groupes styryle, les groupes ester (-CO₂-) et les combinaisons de ceux-ci;
(b) un groupe amino choisi parmi (1) les groupes aminoaryle dans lesquels l'atome d'azote du groupe amino est attaché au noyau de triazine et (2) un groupe amino dans lequel l'atome d'azote du groupe amino est attaché à la fois au noyau de triazine et à l'atome de carbone d'au moins un groupe -CH₂CH₂-; et
(c) un atome d'oxygène.

2. Composé suivant la revendication 1, dans lequel A représente un groupe trihalométhyle.

3. Composé suivant la revendication 2, dans lequel le groupe trihalométhyle est un membre choisi parmi le groupe trichlorométhyle, le groupe tribromométhyle et le groupe triiodométhyle.

4. Composé suivant la revendication 3, dans lequel le groupe trihalométhyle est un membre choisi parmi le groupe trichlorométhyle et le groupe tribromométhyle.

5. Composé suivant l'une quelconque des revendications précédentes, dans lequel le fragment photoinitiateur est choisi parmi un groupe benzoïne, un groupe éther alkylique de benzoïne, un groupe acétophénone, un groupe dialcoxyacétophénone, un groupe benzophénone, un groupe fluorénone, un groupe anthraquinone, un groupe thioxanthone, un groupe triarylsulfonium, un groupe diaryliodonium, un groupe α-acyloxime, un groupe azide, un groupe diazonium, un groupe 3-cétocoumarine, un groupe bisimidazole et un groupe halométhyl-1,3,5-triazine.

6. Composé suivant l'une quelconque des revendications précédentes, dans lequel Y représente A.

7. Composé suivant l'une quelconque des revendications 1 à 5, dans lequel Y représente L-P.

8. Composé suivant l'une quelconque des revendications précédentes, dans lequel R' représente un groupe aryle substitué ou non substitué.

9. Composé suivant l'une quelconque des revendications précédentes, dans lequel R' représente un groupe aromatique hétérocyclique substitué ou non substitué.

10. Composé suivant l'une quelconque des revendications 1 à 8, dans lequel R' représente un groupe alcényle substitué ou non substitué.

11. Composition radiosensible comprenant :
(1) un composé polymérisable, éthyléniquement insaturé, et
(2) un composé suivant l'une quelconque des revendications précédentes.
